# EUROPÄISCHE PATENTSCHRIFT

(11) **EP 1 282 598 B1**
(45) Veröffentlichungstag und Bekanntmachung des Hinweises auf die Patenterteilung: **20.10.2004**
(21) Anmeldenummer: 01947223.2
(22) Anmeldetag: 17.04.2001
(51) Int. Cl.: C07C 315/00, C07C 315/04, C07C 317/44

(54) **VERFAHREN ZUR HERSTELLUNG VON SULFONYL-BENZOYLGUANIDINIUM-SALZEN**
METHOD FOR PRODUCING SULFONYL-BENZOYL GUANIDINIUM SALTS
PROCEDE POUR LA PRODUCTION DE SELS DE SULFONYL-BENZOYL GUANIDINIUM

(30) Priorität: 12.05.2000 DE 10023405
(43) Veröffentlichungstag der Anmeldung: 12.02.2003
(73) Patentinhaber: MERCK PATENT GmbH, 64293 Darmstadt (DE)
(72) Erfinder: BARTMANN, Ekkehard, 64390 Erzhausen (DE); KIRSCHBAUM, Michael, 64331 Weiterstadt (DE)
(86) Internationale Anmeldenummer: PCT/EP2001/004294
(87) Internationale Veröffentlichungsnummer: WO 2001/085679

(56) Entgegenhaltungen:
- EP-A- 0 758 644
- A. ULMAN, ET AL.: "Novel synthesis of 4-[alkyl(aryl)sulphonyl]benzaldehydes: alkyl(aryl)sulphinate anion as a nucleophile in aromatic substitutions" JOURNAL OF ORGANIC CHEMISTRY, Bd. 54, Nr. 19, 15. September 1989 (1989-09-15), Seiten 4691-4692, XP002160704 American Chemical Society, Washington, DC, US ISSN: 0022-3263
- M. BAUMGARTH, ET AL.: "(2-Methyl-5-(methylsulphonyl)benzoyl)- guanidine Na+/H+ antiporter inhibitors " JOURNAL OF MEDICINAL CHEMISTRY, Bd. 40, Nr. 13, 20. Juni 1997 (1997-06-20), Seiten 2017-2034, XP000907364 American Chemical Society, Washington, DC, US ISSN: 0022-2623

## Beschreibung

Die Erfindung betrifft ein Verfahren zur Herstellung von Säureadditions-Salzen der Verbindungen der Formel I, 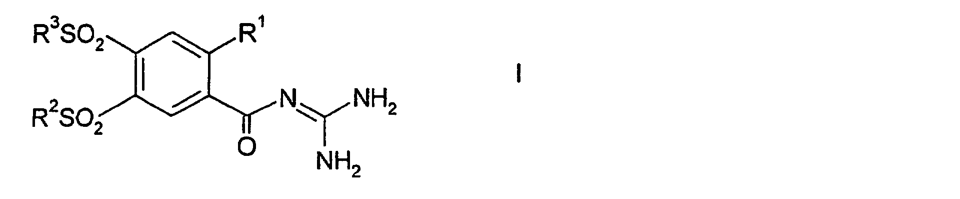 worin R¹, R² und R³ unabhängig voneinander Alkyl mit 1 bis 12 C-Atomen bedeuten, dadurch gekennzeichnet, daß man in einem Schritt A die Verbindungen der Formel II 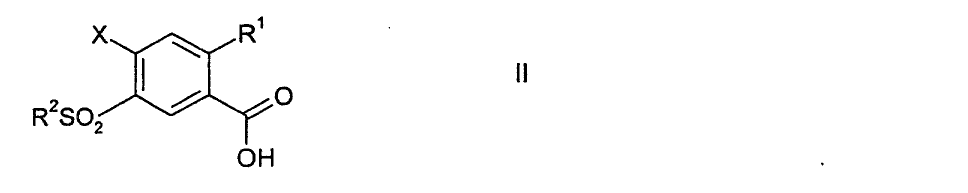 worin R¹ und R² die oben angegebene Bedeutung aufweisen und X F, Cl, Br, Alkyl-oder Arylsulfonat oder Perfluoralkylsulfonat bedeuten, nach üblichen Methoden in die Ester der Formel III 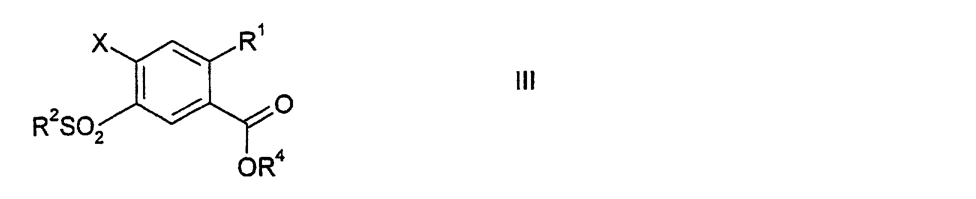 worin R¹, R² und X die oben angegebene Bedeutung aufweisen und R⁴ Alkyl mit 1 bis 10 C-Atomen bedeutet, überführt und diese in einem Schritt B in Gegenwart von Alkylsulfinat zu den Verbindungen der Formel IV 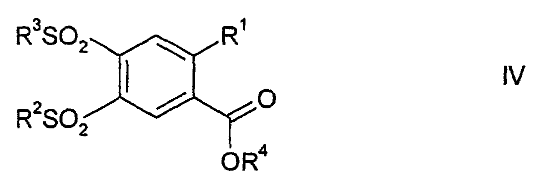 worin R¹, R², R³ und R⁴ die oben angegebene Bedeutung aufweisen, umsetzt, die so erhaltenen Verbindungen der Formel IV in einem Schritt C durch Umsetzung mit Guanidin in die entsprechenden Verbindungen der Formel I überführt und diese in einem Schritt D zur Bildung des Säureadditions-Salzes mit einer geeigneten Säure behandelt.

Sulfonyl-benzoylguanidine sind bekannt und beispielsweise beschrieben in EP 0 758 644 A1. Bei diesen Substanzen handelt es sich um Inhibitoren des zellulären Na⁺/H⁺-Antiproters, d.h. um Wirkstoffe, die den Na⁺/H⁺-Austauschmechanismus der Zellen hemmen (Düsing et al., Med. Klin. 1992, 87, 367-384) und die somit gute Antiarrythmika darstellen, die sich insbesondere zur Behandlung von Arrythmien eignen, die als Folge von Sauerstoffmangel auftreten.

Diese Substanzen zeigen eine gute kardioprotektive Wirkung und eignen sich daher besonders zur Behandlung des akuten Myokardinfarkts, der Infarkt-Prophylaxe, Post-Infarktbehandlung, chronischer Herzinsuffizienz und zur Behandlung von Angina pectoris. Femer wirken sie allen pathologischen hypoxischen und ischämischen Schädigungen entgegen, so daß die dadurch primär oder sekundär verursachten Krankheiten behandelt werden können. Diese Wirkstoffe sind ebenfalls für präventive Anwendungen gut geeignet.

Aufgrund der protektiven Wirkung dieser Substanzen bei pathologischen hypoxischen oder ischämischen Situationen resultieren daraus weitere Anwendungsmöglichkeiten bei chrirurgischen Eingriffen zum Schutz zeitweilig minderversorgter Organe, bei Organtransplantationen zum Schutz der entnommenen Organe, bei angioplastischen Gefäß- oder Herzeingriffen, bei Ischämien des Nervensystems, bei der Therapie von Schockzuständen und zur Verhinderung der essentiellen Hypertonie.

Ferner können diese Verbindungen auch als Therapeutika bei durch Zellproliferation bedingten Erkrankungen wie Arteriosklerose, Diabetes und diabetischen Spätkomplikationen, Tumorerkrankungen, fibrotischen Erkrankungen, insbesondere von Lunge, Leber und Nieren sowie Organhypertrophien und -hyperplasien, eingesetzt werden. Darüber hinaus eignen sich die Verbindungen zu diagnostischen Anwendung zur Erkennung von Krankheiten, die von einer gesteigerten Aktivität des Na⁺/H⁺-Antiporters z. B. in Erythrozyten, Thrombozyten oder Leukozyten begleitet werden.

Die Verbindungen können daher Arzneimittelwirkstoffe in der Human- und Veterinärmedizin verwendet werden. Ferner können sie als Zwischenprodukte zur Herstellung weiterer Arzneimittelwirkstoffe Verwendung finden.

Die Verbindungen der Formel I sind beispielsweise herstellbar nach EP 0 758 644. Die bisher bekannten Synthesen beruhen auf der Einführung von Alkylsulfon-Gruppen in den Ring einer entsprechenden aromatischen Carbonsäure und umfassen eine Vielzahl von Einzelschritten mit zum Teil unbefriedigenden Ausbeuten. Weiterhin weisen die bekannten Verfahren für die industrielle Herstellung nachteilige Reaktionsbedingungen auf. So ist beispielsweise die Einführung von Alkylsulfon-Gruppen in den Ring einer aromatischen Carbonsäure durch nucleophile Substitution von geeigneten Abgangsgruppen mit Alkylsulfanen und anschließender Oxidation wegen der extremen und lang nachwirkenden Geruchsbelästigung durch Alkylsulfane problematisch, selbst wenn diese nur in Spuren frei werden.

Bei einer direkten Einführung der Alkylsulfonyl-Gruppe in den Ring einer aromatischen Carbonsäure durch nucleophile Substitution von geeigneten Abgangsgruppen mittels Alkylsulfinat werden auch bei Verwendung hochpolarer Solventien Temperaturen von mindestens 120°C benötigt, um ausreichende Umsetzungsraten zu erzielen. Es wurde festgestellt, daß in diesem Temperaturbereich eine zwar langsame, bei weiter erhöhten Temperaturen aber stark zunehmende Zersetzungsreaktion von hoher Exothermie eintritt. Auf Grund der großen Wärmeentwicklung dieser Zersetzung besteht die Gefahr, daß bei Großansätzen die Temperaturführung der Reaktion außer Kontrolle geraten. Damit scheidet eine Anwendung dieser Reaktion auf industrielle Maßstäbe aus Sicherheitsgründen aus.

Aufgabe der vorliegenden Erfindung war es daher, ein verbessertes Herstellungsverfahren für die Verbindungen der Formel I und deren Säureadditions-Salze bereitzustellen, das die obengenannten problematischen Reaktionsschritte umgeht und zudem bessere Ausbeuten liefert.

Diese Aufgabe wurde gelöst durch das erfindungsgemäße Verfahren mit den Merkmalen des Anspruchs 1. Es wurde überraschend gefunden, daß der Austausch der Abgangsgruppe X bei den Estern der Formel III deutlich schneller bzw. bei tieferer Temperatur abläuft als bei der entsprechenden freien Säure, die bei den Verfahren nach dem Stand der Technik eingesetzt wird, so daß eine erhebliche Ausbeuteverbesserung eintritt. Durch das erfindungsgemäße Verfahren sind daher auch preiswerte Ausgangsverbindungen mit Chlor-Substituenten am aromatischen Ring als Abgangsgruppen mit sehr guten Ergebnissen verwendbar. Ferner wurde gefunden, daß es nach dem erfindungsgemäßen Verfahren möglich ist, die Schritte A und B sowie die Schritte C und D ohne Aufarbeitung der Reaktionsmischungen nacheinander durchzuführen, so daß Ausbeuteverluste und kostenaufwendige Arbeitsschritte vermieden werden können.

In den Verbindungen der Formeln I, II, III und IV weisen die Reste folgende bevorzugte Bedeutungen auf:
R¹, R², R³ und R⁴ bedeuten unabhängig vorzugsweise Methyl, Ethyl, n-Propyl, n-Butyl oder n-Pentyl. Besonders bevorzugt sind Methyl oder Ethyl, insbesondere Methyl.
X bedeutet vorzugsweise F, CF₃SO₂- oder Cl, insbesondere Cl.

Das erfindungsgemäße Verfahren eignet sich insbesondere zur Herstellung von Säureadditions-Salzen von Verbindungen der Formel 1, worin R¹, R² und R³ gleichzeitig eine Methyl-Gruppe bedeuten (Verbindungen der Formel IA). Ein ganz besonders bevorzugtes Säureadditions-Salz ist das Hydrochlorid.

Das Verfahren ist daher insbesondere zur Herstellung der Verbindung der Formel V geeignet:

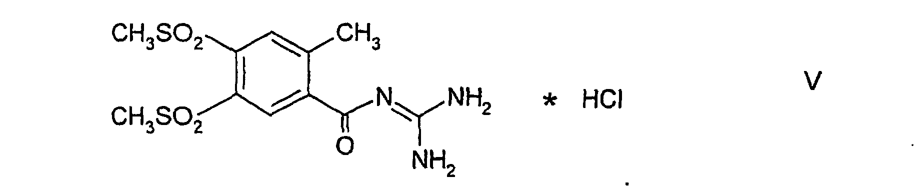

Die Reaktionsdurchführung des erfindungsgemäßen Verfahrens ist einfach, wobei im Schritt A die betreffenden Ausgangsverbindungen der Formel II durch die üblichen literaturbekannten Veresterungsmethoden, wie etwa einer säurekatalysierten Veresterung mit einem entsprechenden Alkohol, wie Methanol oder Ethanol in Gegenwart von überschüssigem Alkohol als Lösungsmittel oder eines geeigneten Cosolvens, der Umsetzung der Carbonsäuresalze von Verbindungen der Formel II mit einem geeigneten Alkylierungsmittel wie z.B. Dialkylsulfat oder der Umsetzung der freien Säuren mit Orthoestern in die entsprechenden Ester überführt werden.

Eine weitere mögliche Veresterungsreaktion ist die Umwandlung der Säure in ein Säurehalogenid und die anschließende Umsetzung mit einem entsprechenden Alkohol zum Ester.

Bevorzugt wird die Veresterung durch Umsetzung der Carbonsäuresalze von Verbindungen der Formel II mit Alkylierungsmitteln wie z.B. Dialkylsulfat oder durch Umsetzung der Verbindungen der Formel II mit einem Orthoester erreicht.

Die Reaktion der Carbonsäuresalze mit Alkylierungsmitteln erfolgt zweckmäßig in der Weise, daß zum jeweiligen, in einem inerten Lösungsmittel gelösten Carbonsäuresalz, das vorzugsweise in situ durch Zugabe einer Base wie z.B. Alkalicarbonat, -hydrogencarbonat, -hydroxid oder -alkoholat, insbesondere eines Alkoholats wie z.B. Kalium-tert-butanolat oder eines Hydroxids wie z.B. Natriumhydroxid, hergestellt wird, vorzugsweise ein Dialkylsulfat zugefügt wird und man die Reaktionspartner bei Raumtemperatur oder erhöhter Temperatur und normalem Druck umsetzt. Dimethylsulfat und Diethylsulfat sind als Alkylierungsmittel besonders bevorzugt.

Vorzugsweise wird die zu veresternde Carbonsäure der Formel II oder deren Salz in einem molaren Verhältnis zum Alkylierungsmittel von 1 zu 1 bis 1 zu 8, insbesondere von 1 zu 2 bis 1 zu 4 eingesetzt.

Besonders bevorzugt wird die Veresterung durch Umsetzung der entsprechenden Säuren mit einem Orthoester wie z.B. Trialkylorthoacetat, Tetraalkylorthocarbonat oder -orthosilicat erzielt. Als Orthoester sind Trimethyl- oder Triethylorthoacetat, Tetramethyl- oder Tetraethylorthocarbonat oder -orthosilicat bevorzugt. Insbesondere ist Tetramethylorthoacetat bevorzugt. Die Veresterungsreaktion wird zweckmäßigerweise bei erhöhten Temperaturen, bevorzugt bei 30-180°C, insbesondere bei 80-120°C in einem inerten Solvens umgesetzt.

Vorzugsweise wird die zu veresternde Carbonsäure der Formel II in einem molaren Verhältnis zum Orthoester von 1 zu 1 bis 1 zu 5, insbesondere von 1 zu 1,5 bis 1 zu 3 eingesetzt.

Als inertes Solvens für den Schritt A kommen vorzugsweise Amide, wie z.B. Dimethylformamid, Dimethylacetamid, Tetramethylhamstoff, cyclische Harnstoffe, wie z.B. N,N-Dimethylimidazolidinon oder Hexamethylphosphorsäuretriamid oder 1-Methyl-2-pyrrolidon (N-Methylpyrrolidon, NMP) in Frage. Sofern die Veresterung mittels Orthoester erfolgt sind weiterhin Ether, wie z.B. Diethylether, Tetrahydrofuran oder Dioxan, Kohlenwasserstoffe, wie z.B. Toluol, Benzol, Hexan oder Heptan bevorzugt. NMP ist besonders bevorzugt.

Mischungen der genannten Lösungsmittel können ebenfalls Verwendung finden.

Die Dauer der Umsetzung im Schritt A hängt von den gewählten Reaktionsbedingungen ab. In der Regel beträgt die Reaktionsdauer 0.5 Stunden bis 2 Tage, vorzugsweise 1 bis 15 Stunden. Im Schritt B werden die Verbindungen der Formel III vorzugsweise in polar-aprotischen Lösungsmitteln bei Temperaturen, die bevorzugt im Bereich von 30-150°C , vorzugsweise von 50-110°C insbesondere bei 80-90°C liegen mit Alkylsulfinat, vorzugsweise mit Alkalialkylsulfinat, umgesetzt.
Als Alkalialkylsulfinat wird vorzugsweise Natrium- oder Kaliumalkylsulfinat verwendet, insbesondere Natriummethylsulfinat oder Kaliummethylsulfinat.

Vorzugsweise werden die Verbindungen der Formel III in einem molaren Verhältnis zum Alkalialkylsulfinat von 1 zu 1 bis 1 zu 4, insbesondere von 1 zu 1,5 bis 1 zu 3 eingesetzt.

Als polar-aprotisches Solvens für den Schritt B kommen vorzugsweise Dimethylsulfoxid, Sulfolan (Tetrahydrothiophen-1,1-dioxid), Dimethylformamid, Dimethylacetamid, Tetramethylharnstoff, cyclische Harnstoffe, wie z.B. N,N-Dimethylimidazolidinon oder Hexamethylphosphorsäuretriamid oder N-Methylpyrrolidon (NMP) in Frage. NMP ist besonders bevorzugt.
Mischungen der genannten Lösungsmittel können ebenfalls Verwendung finden.

Die Dauer der Umsetzung im Schritt B hängt von den gewählten Reaktionsbedingungen ab. In der Regel beträgt die Reaktionsdauer 0.5 Stunden bis 2 Tage, vorzugsweise 1 bis 25 Stunden.

In einer besonders bevorzugten Ausführungsform der Erfindung kann der Schritt B ohne Aufarbeitung des Reaktionsgemisches im Anschluß an den Schritt A durchgeführt werden. Hierzu wird die Veresterung der Verbindungen der Formel II durch Umsetzung mit Alkylierungsmitteln oder vorzugsweise durch Umsetzung mit Orthoestem in einem polaren aprotischen Lösungsmittel, das auch für Schritt B verwendet werden kann, vorzugsweise NMP, wie oben beschrieben ausgeführt. Anschließend versetzt man die erhaltene Reaktionsmischung mit Alkylsulfinat und läßt in der zuvor für Schritt B beschriebenen Art und Weise weiter reagieren.

Im Schritt C werden die Verbindungen der Formel IV vorzugsweise in einem organischen Lösungsmittel bei Temperaturen von -20 bis +60°C, vorzugsweise bei -10 bis +30°, bei normalem Druck mit Guanidin umgesetzt. Als organisches Lösungsmittel für diesen Schritt werden bevorzugt Ether wie Tetrahydrofuran oder Dioxan oder Alkohole wie Methanol, Ethanol, n-Propanol oder i-Propanol verwendet. Mischungen der genannten Lösungsmittel können ebenfalls Verwendung finden. In einer bevorzugten Ausführungsform der Erfindung wird das Guanidin in einem dieser Lösungsmittel aus seinem Säureadditions-Salz wie z.B. dem Guanidiniumchlorid durch Zugabe einer Base wie z.B. Alkalihydroxid oder - alkoholat, insbesondere Natriummethanolat freigesetzt und anschließend ohne mit den Verbindungen der Formel IV umgesetzt.

Vorzugsweise werden die Verbindungen der Formel IV in einem molaren Verhältnis zum Guanidin von 1 zu 1 bis 1 zu 6, insbesondere von 1 zu 2 bis 1 zu 4 eingesetzt.

Die Dauer der Umsetzung im Schritt C hängt von den gewählten Reaktionsbedingungen ab. In der Regel beträgt die Reaktionsdauer 0.5 Stunden bis 20 Stunden, vorzugsweise 1 bis 5 Stunden.

Im Schritt D wird durch Behandlung der Verbindungen der Formel I mit einer entsprechenden Säure das Säureadditions-Salz gebildet. Als Säuren kommen bevorzugt solche in Betracht, die mit den Verbindungen der Formel I physiologisch unbedenkliche und verträgliche Salze bilden.

Bevorzugt können hierfür anorganische Säuren verwendet werden, z.B. Schwefelsäure, Salpetersäure, Halogenwasserstoffsäuren, wie Chlorwasserstoffsäure oder Bromwasserstoffsäure, Phosphorsäuren wie Orthophosphorsäure, Sulfaminsäure, ferner organische Säuren, insbesondere aliphatische, alicyclische, araliphatische, aromatische oder heterocyclische ein- oder mehrbasige Carbon-, Sulfon- oder Schwefelsäuren, z.B. Ameisensäure, Essigsäure, Propionsäure, Pivalinsäure, Diethylessigsäure, Malonsäure, Bernsteinsäure, Pimelinsäure, Fumarsäure, Maleinsäure, Milchsäure, Weinsäure, Äpfelsäure, Benzoesäure, Salicylsäure, 2- oder 3-Phenylpropionsäure, Citronensäure, Gluconsäure, Ascorbinsäure, Nicotinsäure, Isonicotinsäure, Methan- oder Ethansulfonsäure, Ethandisulfonsäure, 2-Hydroxyethansulfonsäure, Benzolsulfonsäure, p-Toluolsulfonsäure, Naphtalinmono- und disulfonsäuren, Laurylschwefelsäure. Insbesondere ist Chlorwasserstoffsäure bevorzugt.

Die Behandlung mit einer Säure erfolgt vorzugsweise in der Weise, daß die Verbindungen der Formel I in einem Lösungsmittel gelöst und mit einer equimolaren Menge der gasförmigen oder flüssigen Säure oder einer Lösung der Säure in einem geeigneten Lösungsmittel versetzt werden.

In einer bevorzugten Ausführungsform der Erfindung kann Schritt D im Anschuß an den Schritt C ohne vorherige Aufarbeitung des Reaktionsgemisches, d.h. ohne Isolation der Verbindung der Formel I, erfolgen, wobei zu Bildung des Säureadditions-Salzes die entsprechende Säure direkt zu dem durch Schritt C erhaltenen Reaktionsgemisch gegeben wird. Hierbei fällt das Säureadditions-Salz der Verbindungen der Formel I aus der Lösung kristallin aus.

Die Mengen der Lösungsmittel für die einzelnen Schritte A, B, C und D ist nicht kritisch, vorzugsweise können 10 g bis 500 g Lösungsmittel je g der umzusetzenden Verbindungen der Formel I, II, III oder IV zugesetzt werden.

Die Verbindungen der Formeln I, II, III und IV können durch übliche Aufarbeitungsschritte, wie z.B. Wasserzugabe zum Reaktionsgemisch und Extraktion nach Entfernung des Lösungsmittels erhalten werden. Es kann vorteilhaft sein, zur weiteren Reinigung des Produktes eine Destillation oder Kristallisation anzuschließen.

Auch ohne weitere Ausführungsformen wird davon ausgegangen, daß ein Fachmann die obige Beschreibung im weitesten Umfang nutzen kann. Die bevorzugten Ausführungsformen sind deswegen lediglich als beschreibende, keineswegs als in irgendeiner Weise limitierende Offenbarung aufzufassen.

Die folgenden Beispiele sollen die Erfindung erläutern, ohne sie zu begrenzen. Sofern nicht anderes angegeben ist, bedeuten Prozentangaben Gewichtsprozent. Alle Temperaturen sind in Grad Celsius angegeben.

Folgende Abkürzungen werden verwendet:
- THF: Tetrtahydrofuran
- KOtBu: Kalium-tert.-butylat
- RT: Raumtemperatur
- MTBE: Methyl-tert.-butylether
- h: Stunde(n)
- d: Tag(e)

### Beispiel 1

Eine Lösung von 29.5 g 4-Chlor-5-methansulfonyl-2-methylbenzoesäure und 18.39 g Trimethylorthoacetat in 100 ml Dioxan wurde bis zur vollständigen Umsetzung der Säure unter Rückfluß erhitzt. Nach Zugabe von 150 ml Toluol wurden die Lösungsmittel soweit entfernt, daß die Mischung noch rührfähig blieb. Anschließend gab man 150 ml 1-Methyl-2-pyrrolidon (NMP) sowie 15.8 g Natriummethansulfinat hinzu und rührte die Mischung für 5 h bei 80°C. Nach Zugabe von weiteren 5.3 g Natriummethansulfinat wurde für 25 h gerührt. Durch übliche Aufarbeitung wurde 4,5-Bismethansulfonyl-2-methylbenzoesäuremethylester erhalten.

### Beispiel 2

Eine Lösung von 100.0 g 4-Chlor-5-methansulfonyl-2-methylbenzoesäure und 68.1 g Trimethylorthoacetat in 322 ml NMP wurde bis zur vollständigen Umsetzung der Säure unter Rückfluß erhitzt. Nach Abdestillieren des überschüssigen Orthoesters wurden 96.5 g Natriummethansulfinat zugegeben, und die Mischung wurde 18 h bei 90°C gerührt. Durch übliche Aufarbeitung wurde 4,5-Bismethansulfonyl-2-methylbenzoesäuremethylester erhalten.

### Beispiel 3

Zu 50.4 g einer 30 %igen Natriummethylat-Lösung in Methanol wurden 87.4 ml THF unter Rühren zugegeben. Anschließend wurden 28.95 g Guanidiniumchlorid eingetragen. Die Suspension wurde für 2 h bei 16 - 24°C gerührt und auf 10°C abgekühlt, bevor 30.8 g 4,5-Bismethansulfonyl-2-methylbenzoesäuremethylester in die Mischung eingetragen wurden. Nachdem für 1 h bei 10°C gerührt worden war, versetzte man die Mischung mit einer entsprechenden Menge einer Salzsäure-Lösung. Durch übliche Aufarbeitung wurde N-(4,5-Bis-methansulfonyl-2-methylbenzoyl)-guanidiniumchlorid erhalten.

## Patentansprüche

1. Verfahren zur Herstellung von Säureaddlitions-Salzen der Verbindungen der Formel I, 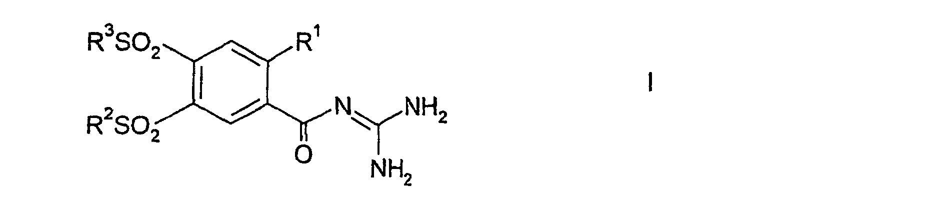 worin R¹, R² und R³ unabhängig voneinander Alkyl mit 1 bis 12 C-Atomen bedeuten, **dadurch gekennzeichnet, daß** man in einem Schritt A die Verbindungen der Formel II 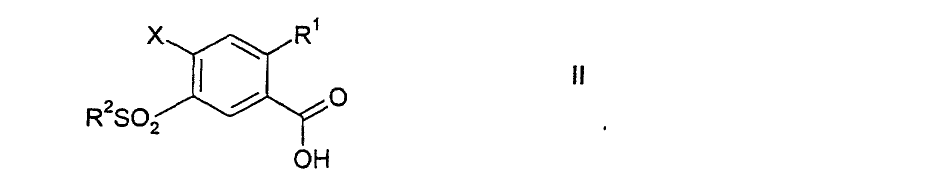 worin R¹ und R² die oben angegebene Bedeutung aufweisen und X F, Cl, Br, Alkyl-oder Arylsulfonat oder Perfluoralkylsulfonat bedeuten, nach üblichen Methoden in die Ester der Formel III 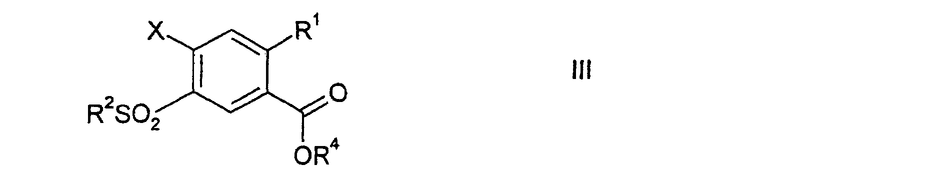 worin R¹, R² und X die oben angegebene Bedeutung aufweisen und R⁴ Alkyl mit 1 bis 10 C-Atomen bedeutet, überführt und diese in einem Schritt B in Gegenwart von Alkylsulfinat zu den Verbindungen der Formel IV 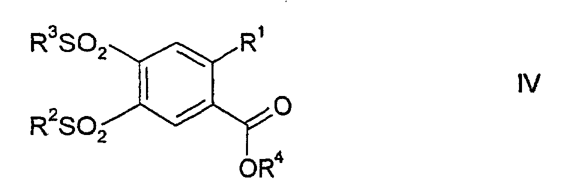 worin R¹, R², R³ und R⁴ die oben angegebene Bedeutung aufweisen, umsetzt, die so erhaltenen Verbindungen der Formel IV in einem Schritt C durch Umsetzung mit Guanidin in die entsprechenden Verbindungen der Formel I überführt und diese in einem Schritt D zur Bildung des Säureadditions-Salzes mit einer geeigneten Säure behandelt.

2. Verfahren nach Anspruch 1, **dadurch gekennzeichnet, daß** Schritt B, die Umsetzung der Verbindungen der Formel III zu den Verbindungen der Formel IV, ohne Aufarbeitung des Reaktionsgemisches im Anschluß an den Schritt A, die Veresterung der Verbindungen der Formel II, durchgeführt wird.

3. Verfahren nach Anspruch 1 oder 2, **dadurch gekennzeichnet, daß** Schritt D, die Behandlung der Verbindungen der Formel I mit einer geeigneten Säure zur Bildung des Säureadditions-Salzes, im Anschuß an den Schritt C, der Umsetzung der Verbindungen der Formel IV zu den Verbindungen der Formel I ohne vorherige Aufarbeitung des Reaktionsgemisches erfolgt.

4. Verfahren nach Anspruch 1, 2 oder 3, **dadurch gekennzeichnet, daß** X in Formel 11 die Bedeutung Cl aufweist.

5. Verfahren nach einem der vorhergehenden Ansprüche, **dadurch gekennzeichnet, daß** im Schritt A die Veresterung durch Umsetzung der entsprechenden Säuren der Formel II mit einem Orthoester erzielt wird.

6. Verfahren nach einem der vorhergehenden Ansprüche, **dadurch gekennzeichnet, daß** für die Schritte A und B 1-Methyl-2-pyrrolidon als Lösungsmittel verwendet wird.

7. Verfahren nach einem der vorhergehenden Ansprüche, **dadurch gekennzeichnet, daß** Schritt B bei einer Temperatur von 50-110°C durchgeführt wird.

8. Verfahren nach einem der vorhergehenden Ansprüche, **dadurch gekennzeichnet, daß** als Säure im Schritt D Chlorwasserstoff-Säure verwendet wird.

9. Verfahren nach einem der vorhergehenden Ansprüche zur Herstellung von Säureadditions-Salzen der Verbindung der Formel IA:

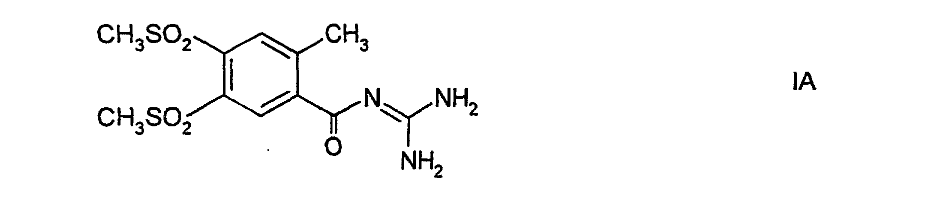

10. Verfahren nach einem der vorhergehenden Ansprüche zur Herstellung der Verbindung der Formel V:

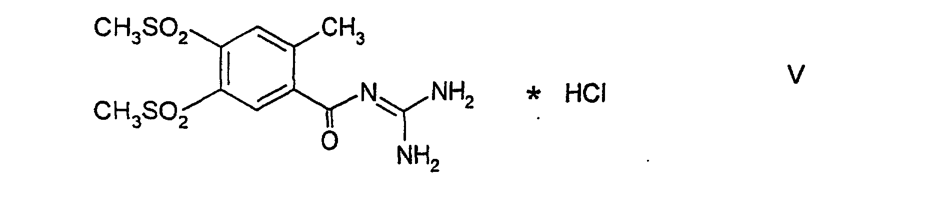

## Claims

1. Process for the preparation of acid-addition salts of the compounds of the formula I 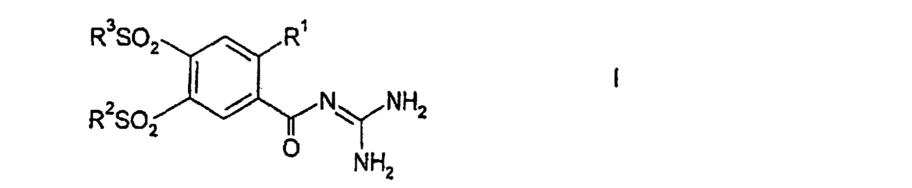 in which R¹, R² and R³, independently of one another, denote alkyl having 1 to 12 C atoms, **characterised in that**, in a step A, the compounds of the formula II 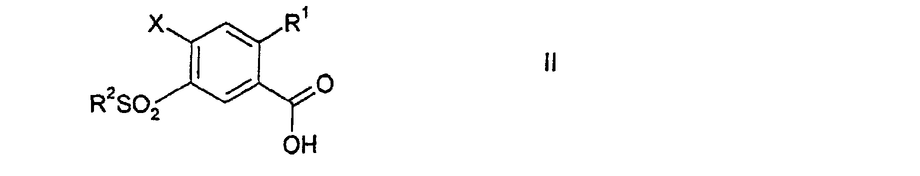 in which R¹ and R² have the meaning indicated above and X denotes F, Cl, Br, alkyl- or arylsulfonate or perfluoroalkylsulfonate, are converted by conventional methods into the esters of the formula III 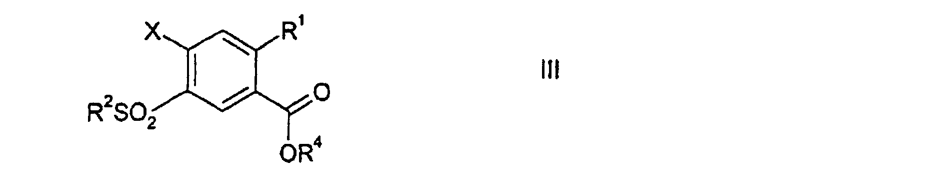 in which R¹, R² and X have the meaning indicated above and R⁴ denotes alkyl having 1 to 10 C atoms, and, in a step B, these are converted in the presence of alkylsulfinate into the compounds of the formula IV 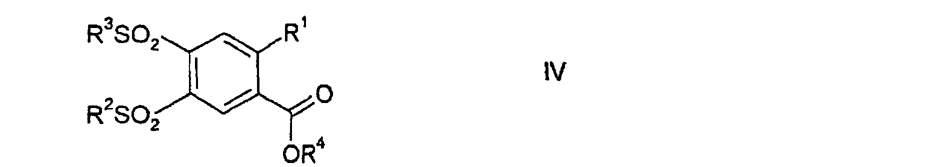 in which R¹, R², R³ and R⁴ have the meaning indicated above, the resultant compounds of the formula IV are, in a step C, converted into the corresponding compounds of the formula I by reaction with guanidine, and, in a step D, these are treated with a suitable acid in order to form the acid-addition salt.

2. Process according to Claim 1, **characterised in that** step B, the conversion of the compounds of the formula III into the compounds of the formula IV, is carried out without work-up of the reaction mixture after step A, the esterification of the compounds of the formula II.

3. Process according to Claim 1 or 2, **characterised in that** step D, the treatment of the compounds of the formula I with a suitable acid in order to form the acid-addition salt, is carried out after step C, the conversion of the compounds of the formula IV into the compounds of the formula I, without prior work-up of the reaction mixture.

4. Process according to Claim 1, 2 or 3, **characterised in that** X in the formula II has the meaning Cl.

5. Process according to one of the preceding claims, **characterised in that** the esterification in step A is achieved by reaction of the corresponding acids of the formula II with an orthoester.

6. Process according to one of the preceding claims, **characterised in that** 1-methyl-2-pyrrolidone is used as solvent for steps A and B.

7. Process according to one of the preceding claims, **characterised in that** step B is carried out at a temperature of 50-110°C.

8. Process according to one of the preceding claims, **characterised in that** the acid used in step D is hydrochloric acid.

9. Process according to one of the preceding claims for the preparation of acid-addition salts of the compound of the formula IA:

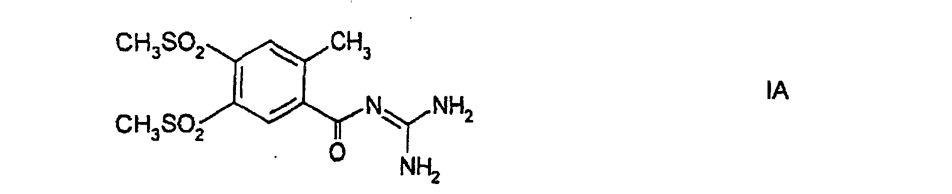

10. Process according to one of the preceding claims for the preparation of the compound of the formula V:

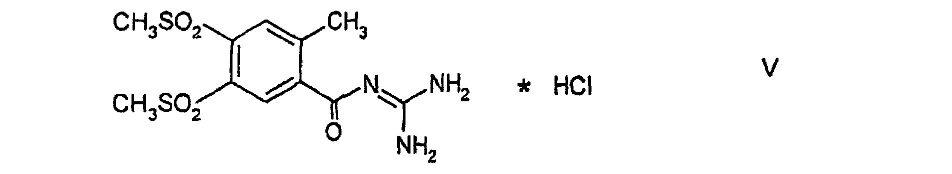

## Revendications

1. Procédé pour la préparation de sels par addition d'acide des composés de la formule I 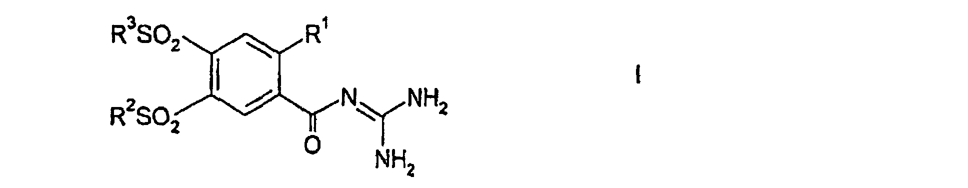 dans laquelle R¹, R² et R³, de manière indépendante les uns des autres, représentent alkyle comportant de 1 à 12 atomes de C, **caractérisé en ce que**, au niveau d'une étape A, les composés de la formule II 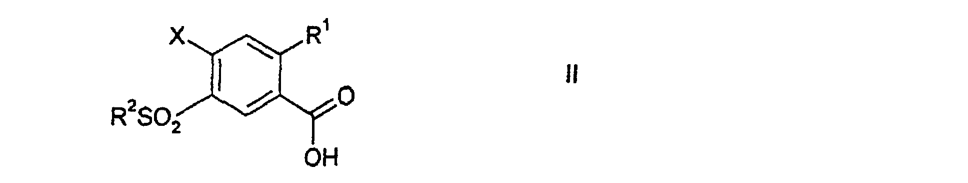 dans laquelle R¹ et R² présentent la signification indiquée ci-avant et X représente F, Cl, Br, alkyl- ou arylsulfonate ou perfluoroalkylsulfonate, sont convertis au moyen de procédés classiques selon les esters de la formule III 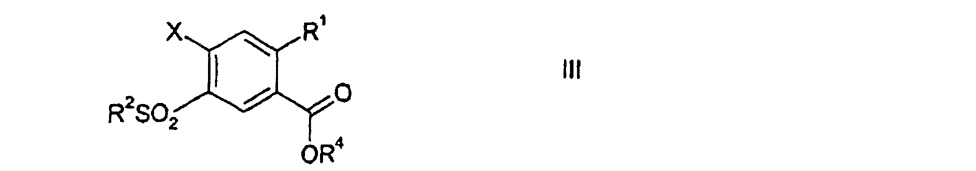 dans laquelle R¹, R² et X présentent la signification indiquée ci-avant et R⁴ représente alkyle comportant de 1 à 10 atomes de C, et, au niveau d'une étape B, ces esters sont convertis en presence d'alkylsulfinate selon les composés de la formule IV 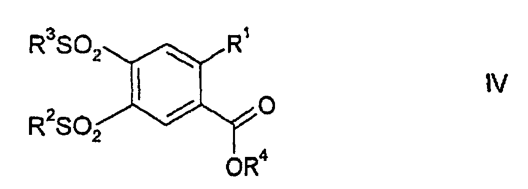 dans laquelle R¹, R², R³ et R⁴ présentent la signification indiquée ci-avant, les composés résultants de la formule IV sont, au niveau d'une étape C, convertis selon les composés correspondants de la formule I par réaction avec guanidine, et, au niveau d'une étape D, ces composés sont traités à l'aide d'un acide approprié afin de former le sel par addition d'acide.

2. Procédé selon la revendication 1, **caractérisé en ce que** l'étape B, soit la conversion des composés de la formule III selon les composés de la formule IV, est mise en oeuvre sans élaboration du mélange de réaction après l'étape A, soit l'estérification des composés de la formule II.

3. Procédé selon la revendication 1 ou 2, **caractérisé en ce que** l'étape D, soit le traitement des composés de la formule I à l'aide d'un acide approprié afin de former le sel par addition d'acide, est mise en oeuvre après l'étape C, soit la conversion des composés de la formule IV selon les composés de la formule I, sans élaboration antérieure du mélange de réaction.

4. Procédé selon la revendication 1, 2 ou 3, **caractérisé en ce que** X dans la formule II présente la signification Cl.

5. Procédé selon l'une quelconque des revendications précédentes, **caractérisé en ce que** l'estérification au niveau de l'étape A est réalisée au moyen d'une réaction des acides correspondants de la formule II avec un orthoester.

6. Procédé selon l'une quelconque des revendications précédentes, **caractérisé en ce que** 1-méthyl-2-pyrrolidone est utilisé en tant que solvant pour les étapes A et B.

7. Procédé selon l'une quelconque des revendications précédentes, **caractérisé en ce que** l'étape B est mise en oeuvre à une température de 50-110°C.

8. Procédé selon l'une quelconque des revendications précédentes, **caractérisé en ce que** l'acide utilisé au niveau de l'étape D est de l'acide chlorhydrique.

9. Procédé selon l'une quelconque des revendications précédentes pour la préparation de sels par addition d'acide du composé de la formule IA:

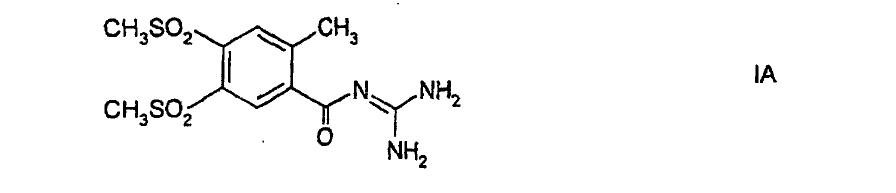

10. Procédé selon l'une quelconque des revendications précédentes pour la préparation du composé de la formule V:
